# FASCICULE DE BREVET EUROPEEN

(11) **EP 3 946 049 B1**
(45) Date de publication et mention de la délivrance du brevet: **19.06.2024**
(21) Numéro de dépôt: 20714223.3
(22) Date de dépôt: 30.03.2020
(51) Int. Cl.: A61B 5/11, A61B 5/00

(54) **DISPOSITIF ET PROCÉDÉ DE BALLISTOCARDIOGRAPHIE**
BALLISTOKARDIOGRAPHIEVORRICHTUNG UND VERFAHREN
BALLISTOCARDIOGRAPHY DEVICE AND METHOD

(30) Priorité: 28.03.2019 FR 1903297
(43) Date de publication de la demande: 09.02.2022
(73) Titulaire: Paris Sciences et Lettres, 75006 Paris (FR); Ecole Pratique des Hautes Etudes, 75014 Paris (FR)
(72) Inventeur: CATHELAIN, Guillaume, 92130 ISSY LES MOULINEAUX (FR); JOUEN, François, 14000 CAEN (FR); JAFFRES, Rémy, 29880 PLOUGUERNEAU (FR)
(74) Mandataire: Cornuejols, Marine Sophie
(86) Numéro de dépôt international: PCT/EP2020/058996
(87) Numéro de publication internationale: WO 2020/193812

(56) Documents cités:
- WO-A1-98/19596
- DE-U1-202008 018 439
- US-A1- 2010 016 685
- US-A1- 2014 371 635
- US-A1- 2015 351 693

## Description

### DOMAINE TECHNIQUE DE L'INVENTION

La présente invention vise un dispositif et un procédé de ballistocardiographie. Elle s'applique, notamment, à la ballistocardiographie, c'est-à-dire à la mesure non intrusive de l'activité cardiaque mécanique.

### ÉTAT DE LA TECHNIQUE

Les textiles et matériaux de support utilisés pour la ballistocardiographie sont élastiques et/ou visqueux pour le confort de l'individu et se déforment de manière isotrope lorsqu'ils sont soumis à une sollicitation mécanique. La déformation ou pression est mesurée, pour le confort de l'individu, à distance de la zone de sollicitation, c'est à dire de la surface de contact entre l'individu et son support mécanique. Le support mécanique, par exemple un matelas, se déforme lors du passage du sang dans une artère : soit dans la direction normale à cette surface, soit dans la direction tangente à cette surface.

Cette distance entre la zone de sollicitation et le point de mesure produit une faible densité d'énergie de déformation/de pression donc l'amplitude et le rapport signal à bruit du ballistocardiogramme sont faibles. En effet, un effet de cône, qui dépend de l'élasticité et de la viscosité du support mécanique, se produit : plus la distance entre le point de mesure et la zone de contact augmente, plus la densité d'énergie de déformation/compression diminue.

Ce phénomène de diffusion se produit en particulier sur les mousses et les textiles. Dans l'objectif de mesurer un ballistocardiogramme d'amplitude et de rapport signal à bruit suffisant pour la détection de l'activité cardiaque, les concepteurs et fabricants utilisent des capteurs très sensibles, des étages de filtrage analogique et des convertisseurs analogiques numériques de très haute résolution ce qui a pour inconvénient un coût élevé et un rapport signal à bruit parfois insuffisant pour le traitement numérique du signal.

Par ailleurs, à la différence d'autres dispositifs de mesures cardiaques tels que les électrocardiogrammes ou les oxymètres de pouls, aucun protocole de mesure n'est correctement établi, car selon l'environnement mécanique de l'individu, les densités de pression et de déformation mesurées sont très différentes. Pour une application dans le domaine de la literie intelligente, il faudrait donc spécifier chaque ballistocardiogramme par la technologie de literie, la technologie du ou des capteur(s), ainsi que la position du ou des capteur(s) sur ou dans la literie, ce qui rendrait caduque l'utilisation de ce dispositif en particulier en milieu médical où les mesures doivent être répétables de manière identique.

Les ballistocardiogrammes sont mesurés actuellement par plusieurs technologies de capteur : les capteurs de pression ou les capteurs de déplacement.

Ces capteurs peuvent être intégrés dans un lit. Ils mesurent des ballistocardiogrammes non répétables puisque les amplitudes varient selon l'environnement mécanique de l'individu. De plus, le rapport signal à bruit est parfois insuffisant malgré une chaîne d'acquisition de haute performance.

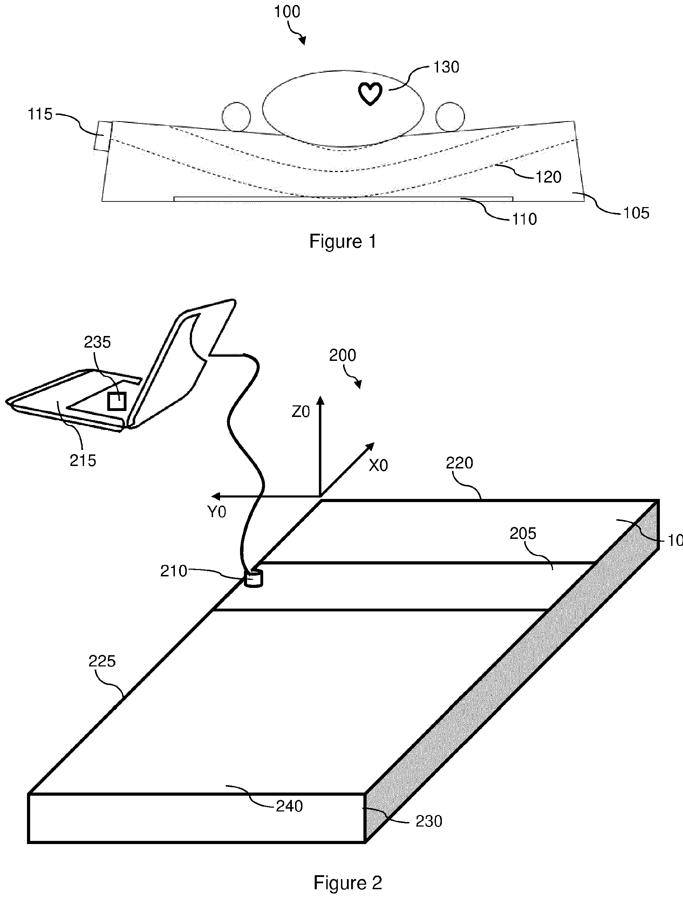

La figure 1 représente schématiquement les dispositifs existants. Un utilisateur 130 (vu en coupe) est allongé sur un matelas 105. Le matelas 105 est positionné sur un sommier muni d'un capteur de contrainte 110 et/ou le matelas comporte un capteur d'inclinaison et/ou de déplacement 115. Les capteurs captent une déformation ou une inclinaison liée à des mouvements quasi statiques 120 provoqués par la respiration et les battements du coeur de l'utilisateur 130. US2014371635 décrit un dispositif existant.

### OBJET DE L'INVENTION

La présente invention vise à remédier à tout ou partie de ces inconvénients.

À cet effet, selon un premier aspect, la présente invention vise un dispositif de ballistocardiographie qui comporte :
- un support non homogène et anisotrope présentant, une partie formant un guide de contraintes ou de déformations, et une partie transmettant moins de contraintes ou de déformations dans le domaine des fréquences entre 0,05Hz et 25 Hz et
- au moins un capteur d'un signal représentatif d'au moins un déplacement et/ou une variation de contrainte quasi statique dudit guide dans le domaine des fréquences entre 0,05 Hz et 25 hertz, positionné en regard dudit guide de contraintes ou de déformations ;
dans lequel le guide de contraintes ou de déformations est surfacique. Le guide de contraintes ou de déformations recouvre au moins partiellement le support. Un élément est surfacique si une dimension est au moins dix fois, préférentiellement trente fois et, encore plus préférentiellement cent fois inférieure à deux autres dimensions d'un repère orthonormé.

Grâce à ces dispositions, la mesure d'un ballistocardiogramme est obtenue à haute résolution et de manière répétable, quelle que soit la nature du support mécanique de l'individu, sans nuire à son confort. L'élasticité et la viscosité du matériau de support sont diminuées, la diffusion des énergies de déformation/compression par effet de cône dans la direction du capteur est également diminuée.

Il est également possible de placer le capteur sur un côté du support ou loin du thorax ou sur la face sur laquelle repose un utilisateur. Par exemple, le capteur peut être placé dans un coin ou au niveau des pieds de l'utilisateur, loin du thorax, pour ne pas gêner l'utilisateur.

De plus, le dispositif est moins coûteux que les solutions connues, car les spécifications techniques du capteur peuvent être plus faibles sans affecter le ballistocardiogramme obtenu.

Dans des modes de réalisation, :
- le guide de contraintes ou de déformations présente une tension sur sa longueur,
- le dispositif comporte un moyen de serrage sous tension du guide de contraintes ou de déformations autour du support,
- le dispositif comporte un moyen de fixation à une partie rigide du support,
- le moyen de serrage ou de fixation ne couvre pas toute la largeur du guide de contraintes ou de déformations et/ou
- le dispositif comporte un réceptacle pour un terminal portable communiquant, tel un intelliphone ou une tablette numérique.

Dans des modes de réalisation, le guide de contraintes ou de déformations présente un module d'Young supérieur, d'au moins 10 % à la valeur du module d'Young en dehors du guide de contraintes ou de déformations selon au moins une direction.

Ces modes de réalisation permettent de transmettre la contrainte ou la déformation de manière efficace.

Dans des modes de réalisation, le support présente une forme généralement parallélépipédique dont la plus grande dimension est dénommée « longueur », la plus petite dimension est dénommée « épaisseur » et la dimension intermédiaire est dénommée « largeur ».

Ces modes de réalisation permettent au dispositif d'être mis en oeuvre lors du repos de l'utilisateur, par exemple lors du sommeil de l'utilisateur.

Dans des modes de réalisation, au moins un capteur est un capteur d'une inclinaison du guide et le guide de contraintes ou de déformations est positionné selon une direction parallèle à la largeur et passant par une source de contraintes ou de déformations.

L'avantage de ces modes de réalisation est de positionner le capteur sur la surface du guide de contraintes ou de déformations et donc de le remplacer ou de l'enlever plus aisément.

Dans des modes de réalisation, au moins un capteur est un capteur de pression et le guide est positionné dans l'épaisseur du support sous une source de contraintes ou de déformations.

Ces modes de réalisation permettent d'éviter que le capteur soit visible ou qu'il puisse être abimé par rapport à un positionnement sur le support.

Dans des modes de réalisation, le guide de contraintes ou de déformations présente un module d'Young de valeur progressive.

L'avantage de ces modes de réalisation est de rendre l'appui de l'utilisateur sur le guide de déformation plus confortable.

Dans des modes de réalisation, le guide de contraintes ou de déformations comporte au moins un matériau tissé.

Grâce à ces dispositions, le guide de contraintes ou de déformations peut être intégré visuellement et mécaniquement à des matériaux de literie ou de repos.

Dans des modes de réalisation, le dispositif objet de l'invention comporte, de plus, un moyen de traitement de chaque signal capturé par chaque capteur et un moyen de comparaison à au moins un modèle prédéterminé pour en déduire des tendances, des troubles ou des anomalies.

Ces modes de réalisation permettent d'assurer le traitement des données issues de chaque capteur.

Selon un deuxième aspect, la présente invention vise un procédé de ballistocardiographie mettant en oeuvre un dispositif objet de l'invention, qui comporte les étapes suivantes :
- capture d'un signal représentatif d'au moins un déplacement et/ou une variation de contrainte quasi statique produite par un utilisateur et parcourant un support,
- segmentation du signal capturé,
- filtrage d'au moins un segment du signal capturé fournissant un signal représentatif d'une activité cardiaque comportant au moins deux battements cardiaques,
- application d'un modèle à chaque période du signal représentatif d'une activité cardiaque et
- détermination d'une fréquence cardiaque et/ou d'une variabilité de la fréquence cardiaque.

Les buts, avantages et caractéristiques particulières du procédé objet de l'invention étant similaires à ceux du dispositif objet de l'invention, ils ne sont pas rappelés ici.

Également, le signal peut être analysé de manière plus précise puisque les résultats sont cohérents physiologiquement.

De plus, il est possible d'intégrer directement le dispositif, dont les données sont traitées au moyen du procédé objet dans l'invention, dans un matelas ou une literie car le capteur peut être de moindre qualité donc plus économique. Notamment, la densité de bruit de l'accélération peut être plus élevée. Donc, le dispositif objet de l'invention est adapté à l'installation en milieu médical par exemple, sans nécessiter de personnel dédié.

Dans des modes de réalisation, le procédé objet de l'invention comporte une phase de calibrage du modèle qui comporte les étapes suivantes :
- capture d'un signal représentatif d'un déplacement ou d'une variation de contrainte quasi statique produite par un utilisateur et parcourant un support,
- segmentation du signal capturé,
- détection d'une enveloppe et d'au moins une période pour chaque segment de signal,
- calcul d'un centre de chaque enveloppe dans la période,
- superposition de centres de chaque période et
- pour chaque segment du signal, création d'un modèle cardiaque correspondant à la moyenne des points superposés à chaque instant de la période prédéterminée.

Ces modes de réalisation permettent de calibrer l'analyse des signaux issus de chaque capteur du dispositif en fonction de l'utilisateur et du support.

Dans des modes de réalisation, l'étape de filtrage fournit un signal représentatif d'une activité respiratoire, le procédé comportant, de plus une étape de détermination d'une fréquence respiratoire et/ou d'évènements d'apnée/dyspnée en fonction d'au moins un segment de signal représentatif d'une activité respiratoire.

Grâce à ces dispositions, des informations cardiaques et respiratoires sont obtenues à partir de l'analyse d'un même signal.

Dans des modes de réalisation, l'étape de segmentation comporte une étape d'élimination de chaque segment de signal représentatif d'un mouvement de l'utilisateur et/ou d'une absence de l'utilisateur sur le support.

Ces modes de réalisation permettent d'étudier uniquement les moments pendant lesquels l'utilisateur est au repos sur le support.

### BRÈVE DESCRIPTION DES FIGURES

D'autres avantages, buts et caractéristiques particulières de l'invention ressortiront de la description non limitative qui suit d'au moins un mode de réalisation particulier du dispositif et du procédé objets de l'invention, en regard des dessins annexés, dans lesquels :
- La figure 1 représente, schématiquement, l'art antérieur de l'invention,
- Le figure 2 représente, schématiquement, un premier mode de réalisation du dispositif objet de l'invention,
- La figure 3 représente, schématiquement, un deuxième mode de réalisation du dispositif objet de l'invention,
- La figure 4 représente, schématiquement, un troisième mode de réalisation du dispositif objet de l'invention,
- La figure 5 représente, schématiquement, et sous forme d'un logigramme, une première succession d'étapes particulière du procédé objet de l'invention,

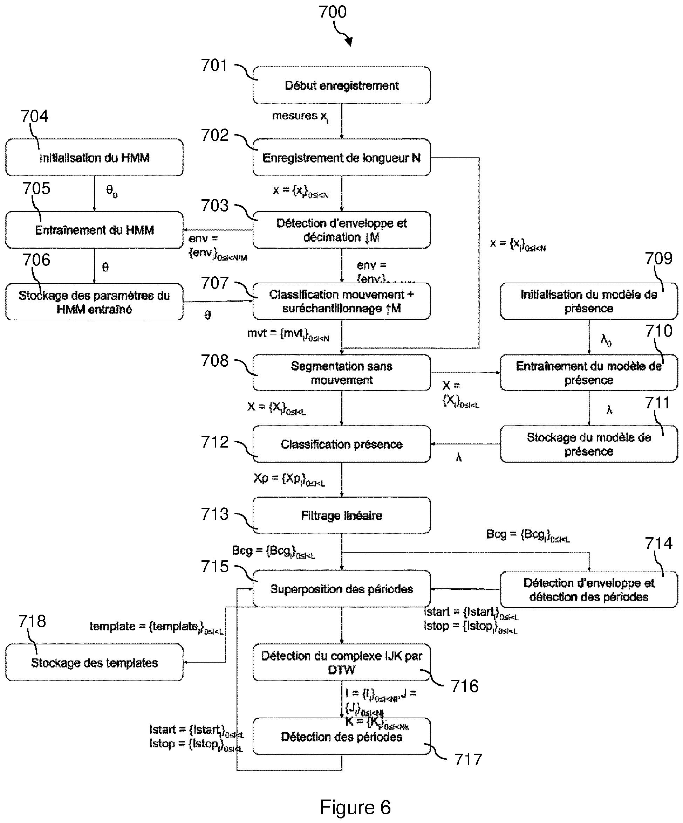

- La figure 6 représente, schématiquement et sous forme d'un logigramme, une deuxième succession d'étapes particulière du procédé objet de l'invention,
- La figure 7 représente, schématiquement, un signal obtenu par un capteur d'un dispositif objet de l'invention,
- La figure 8 représente, schématiquement, un signal après une étape de filtrage d'un procédé objet de l'invention,
- La figure 9 représente, schématiquement, un signal d'une étape d'obtention d'une période d'un procédé objet de l'invention,
- La figure 10 représente, schématiquement, un signal d'une étape de détermination d'une fréquence cardiaque d'un procédé objet de l'invention,
- La figure 11 représente, schématiquement, des signaux lors d'une étape de superposition d'un procédé objet de l'invention,
- La figure 12 représente, schématiquement, un signal après une étape de filtrage d'un procédé objet de l'invention,
- La figure 13 représente, schématiquement, un signal d'une étape de détermination d'une fréquence respiratoire d'un procédé objet de l'invention,
- La figure 14 représente, schématiquement, un modèle obtenu par un procédé objet de l'invention,
- La figure 15 représente des étapes d'un algorithme de détection des battements cardiaques,
- La figure 16 représente des indices de performance,
- La figure 17 représente, schématiquement, une première variante du mode de réalisation illustré en figure 2 et
- La figure 18 représente, en vue de dessus partielle, une deuxième variante du mode de réalisation illustré en figure 2.

### DESCRIPTION DES MODES DE RÉALISATION

La présente description est donnée à titre non limitatif, chaque caractéristique d'un mode de réalisation pouvant être combinée à toute autre caractéristique de tout autre mode de réalisation de manière avantageuse.

On note dès à présent que les figures ne sont pas à l'échelle.

On rappelle ici les définitions suivantes :
- une direction en géométrie est une classe d'équivalence définie dans un ensemble de droites ou de plans par la relation de parallélisme,
- un milieu non homogène est un milieu pour lequel les propriétés du milieu ne sont pas les mêmes en tout point du milieu,
- un milieu anisotrope est un milieu dont les propriétés dépendent de la direction,
- le module de Young ou module d'élasticité (longitudinale) ou encore module de traction est la constante qui relie la contrainte de traction (ou de compression) et le début de la déformation d'un matériau élastique isotrope et
- le tenseur des rigidités généralise le module de Young aux matériaux anisotropes.

Dans la suite du texte, « module de Young » désigne tant le tenseur des rigidités d'un matériau anisotrope que le module de Young d'un matériau isotrope.

On observe, sur la figure 2, qui n'est pas à l'échelle, une vue schématique d'un premier mode de réalisation d'un dispositif objet de l'invention.

Le dispositif de ballistocardiographie 200 comporte :
- un support 105 non homogène et anisotrope comportant une partie formant un guide 205 de contraintes ou de déformations et une partie 240 transmettant moins de contraintes ou de déformations dans le domaine des fréquences entre 0,05Hz et 25 Hz et
- au moins un capteur 210 fournissant un signal représentatif d'au moins un déplacement et/ou une variation de contrainte quasi statique du guide 205 dans le domaine des fréquences entre 0,05 Hz et 25 Hz, positionné en regard du guide 205.

Les fréquences 0,05 Hz à 25 Hz comportent les phénomènes cardiaques et respiratoires.

Préférentiellement, le guide 205 de contraintes ou de déformations présente un module d'Young supérieur, d'au moins 10 %, à la valeur du module d'Young en dehors du guide 205 selon au moins une direction.

Dans les figures 2, 3 et 4, les guides 205, 305 et 410 sont des guides de déformation. Il est plus pertinent de mesurer un déplacement pour les figures 2, 3 et 4 lorsque la surface extérieure du capteur est libre (la contrainte est donc quasiment nulle) et de mesurer une contrainte lorsque le guide est sous tension, le déplacement est donc quasiment nul.

Le support 105 est, par exemple, un matelas en matériau connu de l'homme du métier. Préférentiellement, le support 105 présente une forme généralement parallélépipédique dont la plus grande dimension 225 est dénommée « longueur », la plus petite dimension 230 est dénommée « épaisseur » et la dimension intermédiaire 220 est dénommée « largeur ».

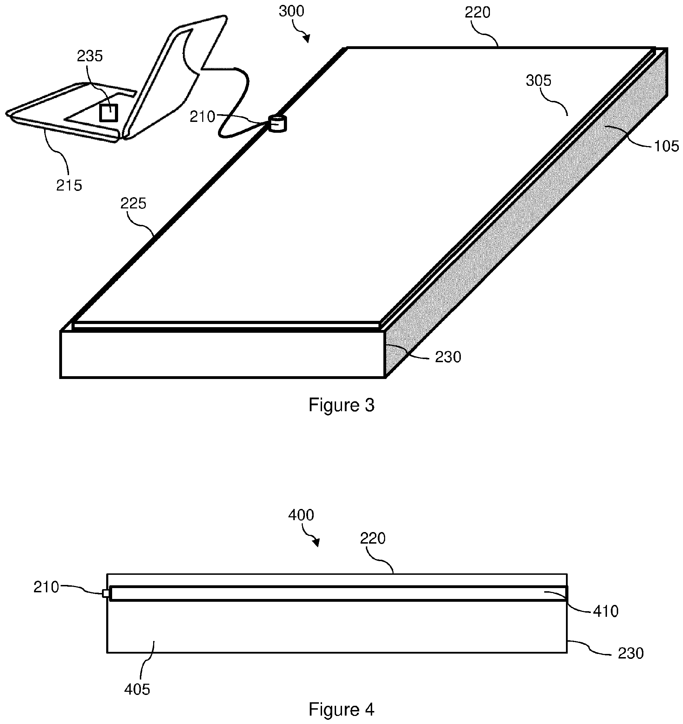

Dans les modes de réalisation représentés en figures 2 et 3, le guide surfacique de déformation, 205 ou 305, est positionné sur le support 105. Dans le mode de réalisation représenté en figure 4, le guide de déformation surfacique 410 est positionné à l'intérieur du support.

Dans le mode de réalisation représenté en figure 2, le guide de déformation 205 est surfacique et recouvre partiellement le support 105. La partie du support 105 recouverte par le guide de déformation 205 correspond à la partie du support 105 sur lequel le thorax d'un utilisateur est positionné lors de l'utilisation du dispositif 200.

On définit un élément surfacique comme un élément dont une dimension est négligeable par rapport aux autres dimensions. En d'autres termes, un élément est surfacique si une dimension est au moins dix fois, préférentiellement trente fois et, encore plus préférentiellement cent fois inférieure à deux autres dimensions d'un repère orthonormé.

Préférentiellement, le guide de déformation 205 est positionné selon une direction parallèle à la largeur et passant par une source de déformation. La source de déformation est le thorax de l'utilisateur animé de mouvements dépendant de la respiration de l'utilisateur ainsi que des battements du coeur de l'utilisateur.

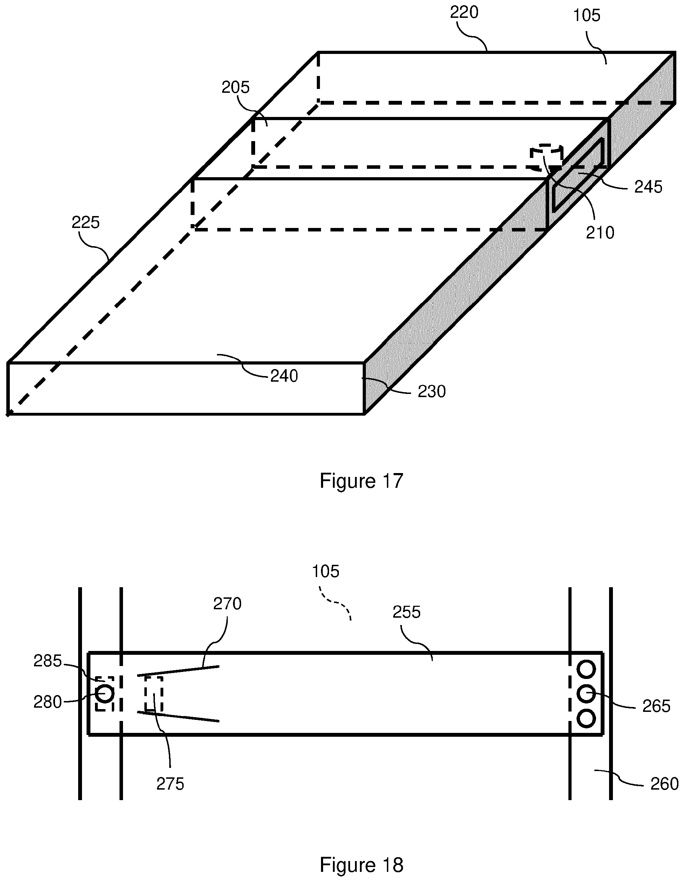

Le guide de déformation 205 est préférentiellement libre de se déplacer par rapport au support 105 pour avoir un coefficient de frottement entre le guide de déformation 205 et le support 105 plus faible. Dans des modes de réalisation, le guide de déformation 205 et/ou le support 105 comportent un moyen de fixation auto agrippant, cousu ou collé. Dans la première variante du mode de réalisation illustré en figure 2 représentée en figure 17, le guide de déformation 205 qui fait le tour du matelas dans sa largeur. Un moyen de fixation 245 fixe deux extrémités de ce guide 205. Dans des variantes, le guide 205 prend la forme d'une bande continue dont chaque segment tient lieu de moyen de fixation. On observe aussi, en figure 17, que le capteur 210 est entre le guide 25 et le matelas 105.

Dans la deuxième variante du mode de réalisation illustré en figure 2 représentée en figure 18, en vue de dessus partielle, le guide 255 est tendu entre des parties rigides 260 du lit, par exemple armature du sommier ou montant du lit, par des moyens de fixation, 265 d'un côté et 280 de l'autre. Préférentiellement, le moyen de fixation le plus proche du capteur 275 ou 285, ici le moyen de fixation 280, est ponctuel, c'est-à-dire couvre une faible partie de la largeur (définie comme pour le support 105) du guide 255, afin que les contraintes et déformations soient concentrées sur ce capteur. On note qu'il peut se former des plis 270 sur le guide 255. Deux positions de capteur (capteur dédié ou intelliphone) sont représentées en figure 18 : en position 275 entre le guide 255 et le support 105 et en position 285 sur la partie rigide 260. Dans les deux variantes illustrées en figures 17 et 18, le guide 205 et 255 est tendu, ce qui favorise la propagation des déformations et contraintes le long de ce guide, c'est-à-dire depuis le thorax de l'utilisateur, source de ces déformations et contraintes, vers le capteur 210, 275 ou 280.

De la même manière, dans la variante représentée en figure 17, préférentiellement le moyen de fixation 245 est ponctuel et ne couvre pas toute la largeur du guide 205 pour concentrer les contraintes et déformations sur le capteur 210 positionné à proximité de ce moyen de fixation 245.

Ainsi, le guide de déformation 205 peut comporter un moyen de serrage, par exemple une boucle de serrage liée à chaque extrémité du guide de déformation pour entourer le support 105. Préférentiellement, le guide surfacique 205 présente une tension sur sa longueur, par exemple par serrage du moyen de serrage ou fixation sous tension du moyen de fixation.

Dans des modes de réalisation, le guide de déformation présente la forme d'une ceinture, préférentiellement tendue entourant le support 105 selon la largeur et l'épaisseur au niveau de l'endroit où l'utilisateur positionne son thorax lorsqu'il utilise le dispositif 200. La ceinture peut être en polymère ou en coton sergé.

La serge est un tissu produit avec l'une des trois armures principales de tissage appelé le sergé. Ainsi, la serge désigne l'ensemble des textiles élaborés par ce type de tissage qui se caractérise par la présence de côtes obliques sur l'endroit et l'envers. Elle peut être à effet chaîne ou trame. C'est une armure dite à décochement.

Le guide de déformation peut être, un matériau homogène ou non, anisotrope ou non. Dans des modes de réalisation, le guide de déformation est un matériau tissé. Le matériau tissé peut être un tissu tridimensionnel connu de l'homme du métier.

Dans des modes de réalisation, le guide de déformation 205 présente un module d'Young de valeur progressive. Par exemple, lorsque le guide de déformation est en tissus, le tissu présente une tension plus élevée au fur et à mesure que l'on s'éloigne de la source de contraintes et de déformations. Dans un autre exemple, le guide de déformation présente un assemblage de rectangles de tissus, ceux proches de la source étant plus élastiques que ceux proches du capteur 210.

Dans le mode de réalisation représenté en figure 2, le capteur 210 est un capteur d'inclinaison ou de déplacement, par exemple un inclinomètre, ou plus généralement basé sur un accéléromètre ou un gyroscope.

Préférentiellement, le capteur 210 est positionné sous le guide 205 et/ou éloigné de la zone de couchage de l'utilisateur, pour éviter de le gêner. Dans des modes de réalisation, le capteur 210 est fixé de manière amovible au guide 205, par exemple au moyen d'un tissu autoadhérant, d'un adhésif, d'une couture ou d'un support aimanté.

Le dispositif 200 comporte, de plus, un moyen de traitement 215 de chaque signal capturé par chaque capteur 210 et un moyen de comparaison 235 comportant au moins un modèle prédéterminé pour en déduire des tendances, des troubles ou des anomalies. Dans des modes de réalisation, le dispositif 200 comporte une carte d'acquisition du signal configurée pour conditionner, filtrer et amplifier la mesure analogique issue du capteur 210.

Dans des modes de réalisation, la densité de bruit d'accélération du capteur est inférieure à 14µg/sqrt(Hz), « sqrt » signifiant la racine carrée. Plus préférentiellement, la densité de bruit d'accélération est inférieure à 90µg/sqrt(Hz).

Un avantage de capteurs de déformation 210 de type accéléromètre par rapport aux capteurs de contraintes est de pouvoir mesurer le ballistocardiogramme selon plusieurs axes, à la différence des capteurs de contraintes monodirectionnels généralement utilisés en ballistocardiographie.

Préférentiellement, le moyen de traitement 215 et le moyen de comparaison 235 sont intégrés à un terminal communicant, et/ou à un serveur applicatif, qui exécute un programme informatique de traitement et de comparaison. Préférentiellement, le programme informatique comporte les étapes du procédé objet de l'invention.

On définit en regard des figures 3 et 4, les caractéristiques géométriques différenciant les modes de réalisation représentés du mode de réalisation représenté en figure 2.

Dans le mode de réalisation représenté en figure 3, le guide de déformation 305 est surfacique et recouvre le support 105 sur une face du support comportant deux axes orthogonaux, l'un étant dans la direction de la largeur, l'autre étant dans la direction de la longueur. Le guide de déformation 305 peut être positionné sur le support 105 à la manière d'un drap-housse ou d'un protège-matelas. Dans des modes de réalisation, le guide de déformation 305 est en coton sergé.

Dans le mode de réalisation représenté en figure 4, le guide de déformation 410, correspond aux modes de réalisations décrits en regard des figures 2 et 3, et placé dans le support 405. Le guide de déformation 410 se situe préférentiellement au moins dans la zone sous laquelle le thorax de l'utilisateur est positionné lorsque le dispositif 400 est utilisé. Dans des modes de réalisation, le guide de déformation 410 est volumique, c'est-à-dire qu'aucune dimension n'est négligeable.

Préférentiellement, le guide de déformation 410 est en textile tridimensionnel connu de l'homme du métier.

Dans le mode de réalisation représenté en figure 4, le capteur 210 est positionné sur une face du support 405 présentant une direction parallèle à l'épaisseur et une direction parallèle à la longueur. Dans des modes de réalisation, le capteur 210 est intégré au support 405, c'est-à-dire que le capteur est inscrit dans le volume défini par le support 405.

Dans des modes de réalisation, un motif de tissage prédéterminé est défini qui permet d'augmenter la transmission de la contrainte ou de la déformation dans le guide.

Dans des modes de réalisation, le guide est en textile tridimensionnel avec des propriétés d'élasticité différentes dans la direction de la largeur selon la latitude. On appelle latitude, une coordonnée d'un point sur le support selon la direction de la largeur.

Dans des modes de réalisation, le guide est en matériau tissé et comporte un assemblage d'au moins deux matériaux tissés.

Dans des modes de réalisation, le capteur est miniaturisé pour mesurer des déformations très locales et pouvoir être attaché à des fibres des textiles.

Dans des modes de réalisation, plusieurs capteurs sont positionnés dans ou sur le même support pour faire de la fusion de données, mieux séparer les sources mécaniques dans le signal et augmenter le rapport signal à bruit.

Dans des modes de réalisation, au moins un capteur est communicant et/ou autonome.

Dans des modes de réalisation, au moins un capteur et le moyen de traitement sont intégrés à un boitier unique.

Dans des modes de réalisation, le dispositif objet de l'invention comporte un réceptacle pour un terminal portable communiquant, tel un intelliphone ou une tablette numérique, comportant un accéléromètre de moindre qualité afin d'amplifier le ballistocardiogramme. C'est-à-dire qu'un capteur du dispositif objet de l'invention est intégré à un terminal portable communiquant. Ces modes de réalisation permettent de mesurer un ballistocardiogramme directement sur un terminal portable communiquant. Aujourd'hui la seule technologie d'analyse du sommeil par intelliphone n'utilise que de l'actigraphie, qui est bien moins efficace que la ballistocardiographie. En actigraphie, il est impossible d'avoir accès aux données de variabilité de fréquence cardiaque qui permet une bonne classification des cycles de sommeil.

Dans des modes de réalisation, le moyen de traitement comporte un moyen d'acquisition analogique-numérique qui met en oeuvre les fonctions suivantes, dans l'ordre :
- application d'un filtre analogique passe-haut,
- amplification,
- application d'un filtre anti-repliement (ou « anti-aliasing » en anglais) et
- une conversion analogique numérique.
Par exemple :
- le filtre analogique passe-haut est un filtre d'ordre un de fréquence de coupure de 0,05 Hz,
- l'amplificateur présente un gain multiplicateur de 500,
- le filtre anti-repliement est un filtre analogique passe-bas d'ordre un de fréquence de coupure égale à la moitié de la fréquence d'échantillonnage et
- le convertisseur code le signal numérique sur au moins douze bits avec un fréquence d'au moins 200 Hz.

Dans des modes de réalisation, le coefficient de frottement entre le support et le guide de déformation est minimisé. Pour le guide de déformation, un capteur de déplacement placé à l'extrémité du guide est utilisé. Plus le coefficient de frottement entre le guide et le support est important, plus l'adhérence est importante et moins le guide de déformation a la liberté de se déformer.

Préférentiellement, le capteur 210 comporte un moyen de communication avec le moyen de traitement 215. Le moyen de communication est, par exemple, un moyen de communication sans fil selon le protocole Bluetooth (marque déposée) ou Zigbee (marque déposée). Dans des modes de réalisation, le capteur comporte un accumulateur rechargeable et un moyen d'optimisation de la chaîne d'énergie.

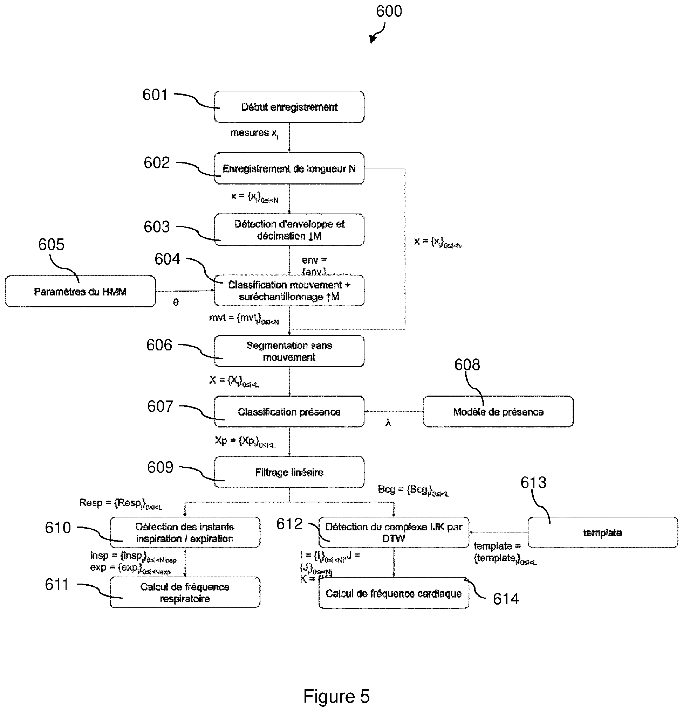

On observe sur la figure 5, un mode de réalisation d'un procédé 600 de ballistocardiographie objet de l'invention.

Le procédé 600 comporte les étapes suivantes :
- capture 601 d'un signal représentatif d'un déplacement ou d'une variation de contrainte quasi statique produite par un utilisateur et parcourant un support sur une première durée prédéterminée,
- segmentation 603 à 608 du signal capturé,
- filtrage 609 d'au moins un segment du signal capturé fournissant un signal représentatif d'une activité cardiaque comportant au moins deux battements cardiaques,
- application 612 d'un modèle à chaque période du signal représentatif d'une activité cardiaque et
- détermination 614 d'une fréquence cardiaque et/ou d'une variabilité de la fréquence cardiaque.

L'étape de capture 601 est préférentiellement réalisée au moyen d'un dispositif 200, 300 ou 400, objets de l'invention. Lors de l'étape de capture 601, le signal correspond à :
- une inclinaison ou déplacement dont les variations sont représentatives de la respiration et des mouvements cardiaques d'un utilisateur sur un support où
- une contrainte dont les variations sont représentatives de la respiration et des mouvements cardiaques d'un utilisateur sur un support.

Le procédé 600 peut être mis en oeuvre sur des signaux issus de plusieurs capteurs dont les résultats sont comparés.

Le signal d'une longueur de N échantillons est enregistré 602 et segmenté. Par exemple, on ajoute un horodatage à chaque échantillon, c'est-à-dire qu'on renseigne l'instant de mesure de cet échantillon. Dans des modes de réalisation, l'étape d'enregistrement 602 est réalisée en continu et préférentiellement en temps réel. Par exemple un microcontrôleur est placé dans le même boitier que le capteur et met en oeuvre le procédé 600. La segmentation peut également être dénommée fenêtrage.

Un segment, ou une fenêtre, est constitué de plusieurs échantillons et peut durer entre une seconde et dix minutes, par exemple. Préférentiellement, la fréquence d'échantillonnage 602 est fixe et comprise entre 200 Hz et 1 kHz.

L'enveloppe est détectée 603 pour chaque segment et décimée. Par exemple, l'enveloppe peut être détectée 603 par application d'une transformée de Hilbert est à chaque segment, la détermination d'une valeur absolue du signal ou de la valeur efficace du signal (« Root Mean Square » en anglais, d'acronyme RMS). La décimation consiste à ne conserver qu'un échantillon sur M, M étant le rapport de décimation. Préférentiellement, M est compris entre 20 et 1000.

Préférentiellement, les étapes de segmentation 603 à 608 comportent une étape d'élimination, 604 et 607, de chaque segment de signal représentatif d'un mouvement de l'utilisateur et/ou d'une absence de l'utilisateur sur le support.

Lors d'une étape 604, un modèle de Markov caché (« Hidden Markov Model » d'acronyme HMM, en anglais) est appliqué. Le modèle de Markov caché, dont les paramètres sont définis à l'étape 605, présente deux états : un état dont les observations correspondent à des mouvements et un état dont les observations correspondent à une absence de mouvement. De plus, lors de l'étape 604 une séquence d'observation correspondant à l'enveloppe du signal 603 est produite.

Une observation est définie comme une valeur d'un signal à un instant prédéterminé : c'est ici l'enveloppe décimée 603 de ce que mesure l'accéléromètre. On définit une séquence d'observation comme une suite d'observations ordonnées dans le temps.

Dans le modèle définit à l'étape 604, on fait l'hypothèse que la séquence d'observation est une variable aléatoire. La séquence des états est synchronisée avec la séquence d'observation. La séquence des états est déduite du modèle de Markov caché grâce à la séquence d'observation par l'algorithme de Viterbi.

Un algorithme de Viterbi, fondé sur le modèle de Markov caché est appliqué à la séquence d'observation pour retrouver la séquence des états qui se cache derrière la séquence d'observation, donc on peut classer 604 le signal en sous-séquences d'observation, certaines séquences correspondant à du mouvement et certaines séquences correspondant à de l'absence de mouvement.

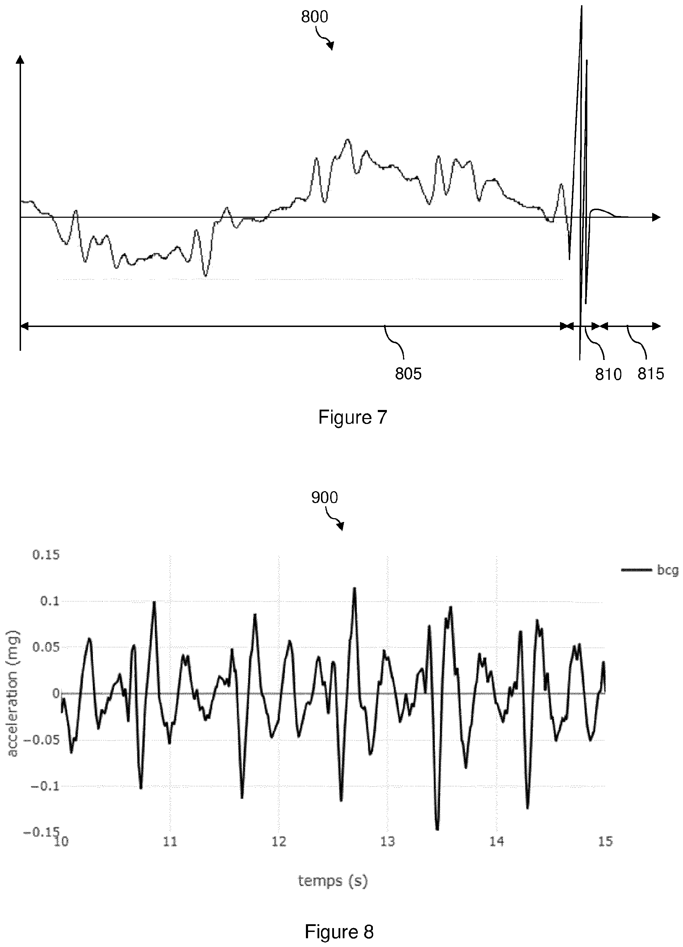

Le mouvement est représenté en figure 7 par un signal 810 de grande amplitude par rapport aux autres grandeurs du signal. En effet, lorsqu'un utilisateur bouge sur le support, les mouvements qu'il effectue provoquent une déformation et/ou une contrainte dont l'ordre de grandeur est au moins cinq fois supérieur à l'ordre de grandeur des déplacements et/ou contraintes appliquées lors de battements cardiaques et de respirations.

Préférentiellement, lors de l'étape de classification 604, un suréchantillonnage est effectué. Le signal enregistré en 602 a un échantillonnage entre 200 et 1000 Hz. L'algorithme classification de Viterbi a une complexité croissante avec le nombre d'échantillons. Pour une meilleure performance du procédé 600, il est préférable, avant d'effectuer la classification, de décimer le signal 603 de telle sorte à obtenir une fréquence d'échantillonnage intermédiaire de 1 à 10 Hz, par exemple 4 Hz. Après la classification, le signal est suréchantillonné, par interpolation linéaire, par exemple avec le même facteur que celui de la décimation. Ainsi, on améliore les performances de l'algorithme de classification tout en conservant la même fréquence d'échantillonnage avant et après la classification.

Une fois la classification effectuée, seuls les signaux, 805 et 815, classifiés comme ne représentant pas un mouvement de l'utilisateur sur le support sont sélectionnés lors d'une étape de segmentation 606.

Puis, un modèle de présence 608 est appliqué aux signaux segmentés lors d'une étape de classification 607 d'un signal en fonction de la présence d'un utilisateur sur le support. En effet, lorsqu'un utilisateur est absent du support, le signal représentatif de cette absence 815 a une amplitude de l'ordre de grandeur de bruit.

Le modèle de présence est obtenu par le procédé 700 de calibration. Le modèle de présence est constitué de deux densités A et B de probabilité gaussiennes, chacune caractérisée par un écart type et une valeur moyenne. La densité A une valeur moyenne et un écart-type élevé : elle correspond à la présence d'un utilisateur sur le support sans mouvement. La densité B a une valeur moyenne et un écart type faible : elle correspond à l'absence d'un utilisateur sur le support.

Pour chaque segment de signal sans mouvement 606, on calcule la valeur moyenne et l'écart type de l'enveloppe du signal : cela revient à considérer une densité de probabilité Ci pour chaque segment 606. On associe cette densité Ci à la densité A ou la densité B la plus proche, la proximité des densités étant ici définie comme une combinaison linéaire de la distance euclidienne entre leur valeur moyenne et la distance euclidienne entre les écart-types. Ainsi, on a classé chaque segment sans mouvement 606 selon la catégorie « segment de présence de l'utilisateur sans mouvement » ou « segment d'absence de l'utilisateur ».

Seuls les segments classifiés représentatifs de la présence d'un utilisateur sur le support sont utilisés dans la suite du procédé 600.

Une étape de filtrage est appliquée auxdits segments 609. Un filtre passe-bande, composé d'un filtre à réponse impulsionnelle infinie passe-bas d'ordre 2, de fréquence de coupure 25 Hz et de facteur de qualité 0.707 et un filtre à réponse impulsionnelle infinie passe-haut d'ordre 2, de fréquence de coupure 5 Hz et de facteur de qualité 0.707 sont appliqués pour obtenir un signal représentatif d'une activité cardiaque.

Le signal représentatif d'une activité cardiaque est illustré en figure 8.

Dans des modes de réalisation préférentiels, l'étape de filtrage 609 fournit un signal représentatif d'une activité respiratoire, le procédé comportant, de plus une étape de détermination, 610 et 611, d'une fréquence respiratoire et/ou d'évènement d'apnée/dyspnée en fonction d'au moins un segment de signal représentatif d'une activité respiratoire. Un filtre à réponse impulsionnelle infinie passe-bas d'ordre 2, de fréquence de coupure 5 Hz et de facteur de qualité 0.707 est appliqué pour obtenir un signal représentatif d'une activité respiratoire.

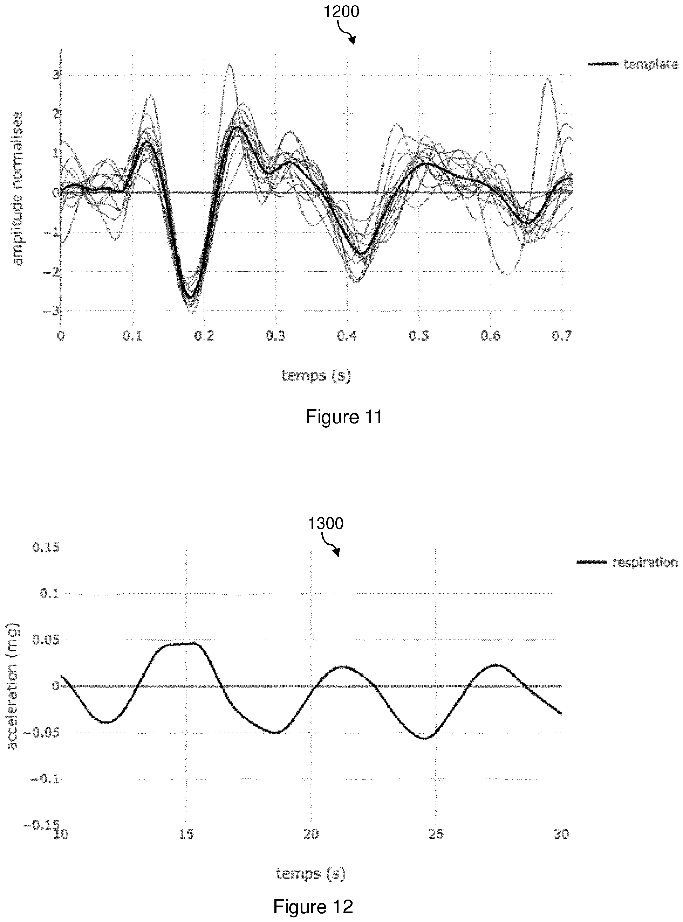

Les signaux représentatifs d'une activité respiratoire sont illustrés en figure 12.

L'étape de détermination d'une fréquence respiratoire comporte une étape de détection des instants d'inspiration et d'expiration 610. Par exemple, lors de l'étape de détection 610, un instant d'inspiration correspond à un minimum local et un instant d'expiration correspond à un maximum local.

L'étape de détermination comporte également une étape de calcul d'une fréquence respiratoire 611. Le calcul de la fréquence est effectué à partir de la période moyenne entre deux instants d'inspiration et/ou d'expiration.

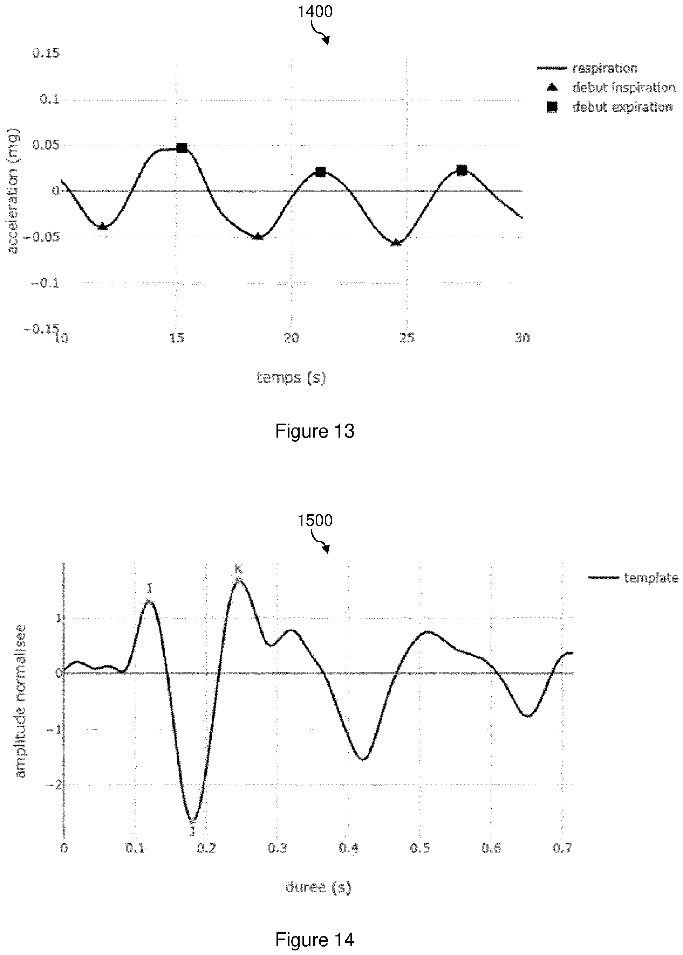

L'étape de détermination d'une fréquence cardiaque comporte, une étape de détection 612 d'un complexe IJK par déformation dynamique temporelle (« Dynamic Time Warping » en anglais, d'acronyme DTW). Le complexe IJK, figure 14, connu en ballistocardiographie, correspond à la systole ventriculaire gauche. Le segment IJ correspond à la contraction du ventricule et le segment JK correspond à la détente du ventricule. On prend le pic J comme référence du battement cardiaque et pour le calcul de la fréquence cardiaque.

Puis, pour chaque segment, on détecte automatiquement, en effectuant une vérification et une correction manuelle si nécessaire, l'amplitude des pics J du ballistocardiogramme et on détermine les valeurs médianes, minimales et maximales de l'amplitude des pics J. Ces éléments statistiques permettent de rendre compte de la qualité de l'éjection ventriculaire gauche.

Lors de l'étape de détection 612, un modèle définit lors de la phase calibrage est appliqué aux segments.

Lors de l'étape de calcul 614 de la fréquence cardiaque, le battement cardiaque moyen de l'utilisateur à partir du modèle appliqué qui minimise la déformation dynamique temporelle. Une fois le modèle appliqué, on détecte les pics J des complexes IJK. On calcule le délai entre deux pics J comme l'intervalle entre chaque battement cardiaque. On fait une interpolation linéaire pour échantillonner l'intervalle entre deux pics J à 1 Hz. On prend l'inverse de cette série et on la multiplie par 60 : on obtient la série de fréquence cardiaque en battement par minute (bpm), échantillonnée à 1Hz après interpolation linéaire.

Préférentiellement, le procédé 600 comporte une phase de calibrage 700 du modèle sur une deuxième durée prédéterminée qui comporte les étapes suivantes :
- capture 701 d'un signal représentatif d'un déplacement ou d'une variation de contrainte quasi statique produite par un utilisateur et parcourant un support,
- segmentation 703 à 712 du signal capturé,
- détection d'une enveloppe 714 et d'au moins une période pour chaque segment de signal,
- calcul d'un centre de chaque enveloppe dans la période,
- superposition 715 de centres de chaque période et
- pour chaque segment du signal, création 718 d'un modèle cardiaque correspondant à la moyenne des points superposés à chaque instant de la période prédéterminée.

L'étape de capture 701, de la phase de calibrage correspond à l'étape de capture 601 du procédé 600.

Les étapes d'enregistrement 702, de détection 703 d'une enveloppe, 707 et classification en fonction d'un mouvement, 708 de segmentation, 712 de classification en fonction d'une présence et 713 de filtrage correspondent respectivement aux étapes 602 à 604, 606, 607 et 609 du procédé 600.

Le modèle de Markov caché 605 utilisé dans le procédé 600 est obtenu après initialisation 704 et entrainement 705 d'un modèle de Markov caché à partir des enveloppes détectées lors de l'étape de détection d'enveloppe 703. Les paramètres du modèle de Markov caché entrainé sont ensuite enregistrés 706 pour être utilisés dans le procédé 600.

Le modèle de présence 608 utilisé dans le procédé 600 est obtenu après initialisation 709 et entrainement 710 du modèle de présence à partir des enveloppes détectées sans mouvement après segmentation 708. Les paramètres du modèle de présence entrainé sont ensuite enregistrés 711 pour être utilisés dans le procédé 600.

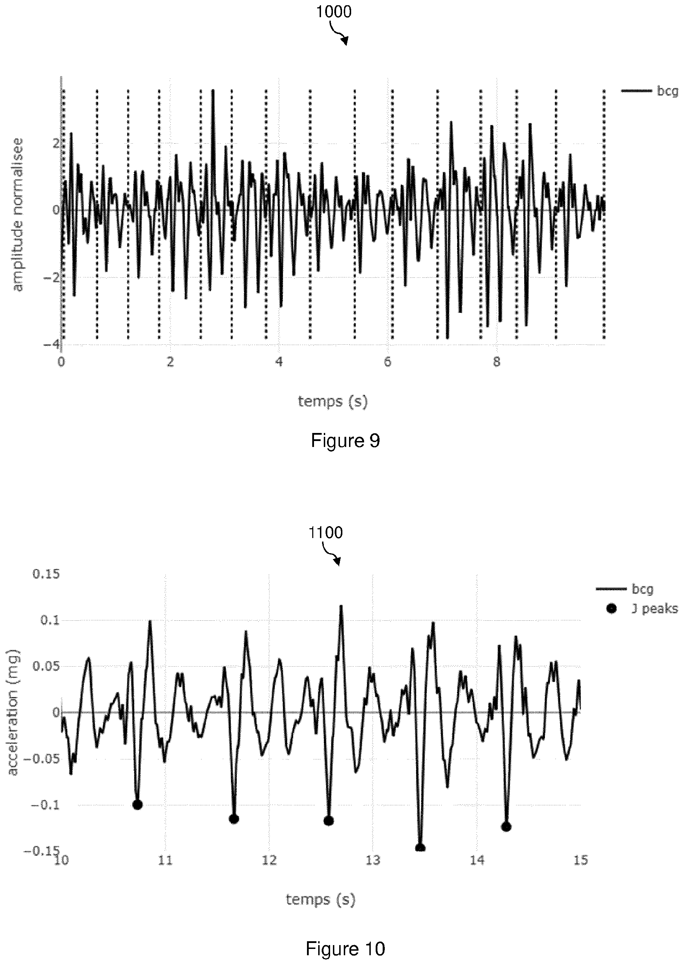

Après l'étape de filtrage 713, on détermine les minima locaux de chaque enveloppe 714 de chaque segment comme représenté en figure 10, ce qui permet de définir des périodes du signal. Les périodes sont illustrées en pointillé en figure 9. On définit le centre de chaque période par la position du minimum global du signal cardiaque dans la période. On superpose 715 ensuite chaque période en positionnant leurs centres sur une référence commune, le centre de la superposition. La superposition des périodes est représentée en figure 11.

Un modèle est ensuite réalisé pour chaque segment de présence et stocké 718 en calculant la moyenne des points des signaux de ces segments superposés.

On effectue une déformation dynamique temporelle 716 (« Dynamic Time Warping » en anglais, d'acronyme DTW) du modèle avec des fenêtres du signal. Une fenêtre du signal présente la durée moyenne d'un battement cardiaque, comportant une contraction et une relaxation mécanique du coeur. A titre d'exemple, la durée moyenne d'un battement cardiaque est comprise entre 0,5 et 1 seconde en général.

Par exemple la figure 11 représente dix fenêtres du signal qui minimisent la distance de déformation dynamique temporelle avec le modèle. La figure 11 a été construite par choix d'une taille de fenêtre, et de la position du pic J dans cette fenêtre. Dans l'exemple représenté en figure 11, la taille de la fenêtre est de 0.72 seconde et la position du pic J dans cette fenêtre à 0.18 seconde. Puis, pour chaque minimum local du signal, on sélectionne une fenêtre de même taille pour que le minimum local du signal soit positionné à 0.18 seconde du début de la fenêtre. Pour chacune de ces fenêtres on calcule la distance de déformation dynamique temporelle, on applique un seuil aux distances de déformation dynamique temporelle : les distances de déformation dynamique temporelle inférieures au seuil correspondent à des battements cardiaques. Les fenêtres ressemblent donc très fort au modèle, ce sont des battements cardiaques. Le modèle, sur lequel un complexe IJK est référencé, est représenté de manière isolée en figure 14.

On superpose 715 les battements cardiaques pour obtenir un nouveau modèle, représentatif de l'enregistrement étudié. Le nouveau modèle est obtenu par superposition des dix battements cardiaques les plus proches du premier modèle. Le modèle est donc plus spécifique que le modèle initial. On a donc adapté un modèle générique à l'enregistrement de l'utilisateur, la position de l'utilisateur et le support sur lequel l'utilisateur repose.

Pour chaque segment de présence, on effectue des itérations de détection 716 du complexe IJK, de détection et superposition des périodes 717 et 715 jusqu'à convergence du modèle de battement cardiaque.

Dans des modes de réalisation, le dispositif objet de l'invention comporte aux moins deux capteurs de contraintes et/ou de déplacements placés selon des longitudes différentes pour correspondre à deux sources différentes. On appelle longitude, la dimension selon un axe dans la direction de la longueur. Par exemple, un capteur de contraintes et/ou de déplacement est placé en regard du thorax de l'utilisateur et un capteur de contraintes et/ou de déplacement est placé en regard, du bassin, des pieds ou de la tête de l'utilisateur.

Dans ces modes de réalisation, le procédé 600 comporte une étape de mesure de la rigidité artérielle par mesure de la vitesse d'onde de pouls (« Pulse Wave Velocity » d'acronyme « PWV » en anglais). L'étape de mesure de la rigidité artérielle comporte une étape de mesure du passage du sang de l'utilisateur à au moins deux endroits où sont positionnés au moins un capteur. On mesure les délais entre les pics J du ballistocardiogramme correspondant au thorax de l'utilisateur et le ballistocardiogramme lié au deuxième emplacement, par exemple les pieds. Puis on calcule la vitesse d'one de pouls en fonction du délai mesuré et de la distance entre les capteurs selon la longueur.

### EXEMPLES

Dans la suite, des tests ont été effectués avec différents guides et une référence comportant uniquement un support. On appelle « face supérieure » la face du support sur laquelle un utilisateur s'allonge.

Dans les exemples ci-dessous, chaque guide de contraintes ou de déformations présente un module d'Young supérieur, d'au moins 10 % à la valeur du module d'Young en dehors du guide de contraintes ou de déformations,

### Support de référence :

On prend comme support de référence 105 un matelas composé de mousse polyuréthane et de latex Malvik (marque déposée) ferme de dimension 200x80 cm ainsi qu'un lit en pin Utaker (marque déposée) disponibles chez Ikea (marque déposée).

La base (X0,Y0,Z0) est orthonormale et fixe par rapport au bâti, par exemple le sol (voir figure 2). Lorsque l'individu est allongé sur le dos :
- l'axe X0 correspond à l'axe tête-pied (la longueur du matelas 105)
- l'axe Y0 correspond à l'axe gauche-droite (la largeur du matelas 105) et
- l'axe Z0 correspond à l'axe dorso-ventral (direction de la gravité) (la hauteur du matelas 105).

On retire la housse du matelas. On attache avec du scotch double-face le capteur directement sur le matelas, centré à la position suivante en prenant comme centre du repère le coin supérieur gauche de la face supérieure du matelas :
- x0 = - 50 cm
- y0=-10cm

Par la suite on conserve ces coordonnées de position du capteur, seul le support et la fixation changeront.

### Exemple A

On ajoute au support de référence 105 une couche de textile tridimensionnelle extraite d'un surmatelas Aerospacer (marque déposée) de la marque Medstrom (marque déposée). La couche de textile tri-dimensionnel est le guide de déformation.

Une couche de textile tridimensionnel a un module d'élasticité anisotrope : le module d'Young dans la direction X0 est inférieur au module d'Young dans la direction Y0.

Le capteur est positionné aux mêmes coordonnées x0 et y0, sur la face supérieure de la couche de textile tridimensionnel que pour la mesure de référence.

### Exemple B

On ajoute au support de référence 105 un ruban en coton Sergé. Le ruban de coton entoure le matelas dans le sens de la largeur, selon la coordonnée y=y0. Le ruban est tendu par une boucle de serrage en polyamide.

Le capteur est scotché par un double-face sur la face supérieure du ruban, sur la position x=x0. Ainsi, le capteur est positionné aux coordonnées x0 et y0 sur la face supérieure du ruban.

### Résultats

On compare les amplitudes des pics J du ballistocardiogramme et les valeurs efficaces du ballistocardiogramme et de l'accélération de respiration, pour chaque support. On réalise trois essais consécutifs pour chaque support, pour s'assurer de la répétabilité des mesures. On indique aussi le bruit ambiant avec un essai en l'absence d'une personne allongée.

Dans la suite, on appelle « performances » l'amplitude de la valeur efficace du ballistocardiogramme capturé, les amplitudes minimum, maximum, médiane ou moyenne des pics J du ballistocardiogramme capturé.

Pour le ballistocardiogramme selon la direction x, on mesure que :
- la valeur efficace du bruit est de l'ordre de huit µg,
- le rapport signal à bruit varie entre quatre et sept décibels selon les configurations et
- les guides des exemples A et B augmentent les performances.
Pour le ballistocardiogramme selon la direction y, on mesure que :
- la valeur efficace du bruit est de l'ordre de neuf µg,
- le rapport signal à bruit varie entre huit et dix décibels selon les configurations et,
- les guides de déformation des exemples A et B augmentent les performances.

On note ici que la valeur efficace du signal représentatif de l'accélération n'est pas suffisante pour caractériser les performances du guide objet de l'invention, et que ce qui compte est surtout l'amplitude des pics J. En particulier, l'amplitude minimale des pics J pour chaque enregistrement est une performance intéressante, le pic J le plus petit est celui qui est le plus difficile à détecter car d'amplitude proche de celle du bruit. Par exemple, le guide de l'exemple A présente une valeur efficace du signal représentatif de l'accélération plus faible que pour le support sans guide mais les amplitudes de pics J sont plus grandes.

Bien qu'ils augmentent généralement les performances, on remarque que les guides de déformation peuvent avoir plusieurs effets sur les performances, selon le support utilisé et selon les axes de déplacement considérés. On constate que le guide de l'exemple B permet d'améliorer grandement les performances jusqu'à 72% d'augmentation de l'amplitude minimale des pics J. Le guide de l'exemple A augmente les performances plutôt sur l'axe x que sur l'axe y.

Lorsque le support est modifié pour comporter un matelas muni d'une housse, on constate que le guide de l'exemple B augmente considérablement l'amplitude minimale des pics J qui peut être jusqu'à vingt-cinq pour cent plus élevée.

Il est très important de distinguer les amplitudes minimales et maximales des pics J. Sur un même enregistrement, l'amplitude des battements cardiaques, et des pics J en particulier, varie avec la respiration. Les pics J de faible amplitude sont les plus difficiles à détecter. La performance la plus intéressante à évaluer est donc l'amplitude minimale des pics J.

Les guides de déformation des exemples A et B permettent d'augmenter l'amplitude minimale des pics J. Par exemple, le guide de l'exemple A augmente de deux à trente-cinq pour cent l'amplitude minimale des pics J et le guide de l'exemple B augmente de douze à soixante-douze pour cent l'amplitude minimale des pics J.

On voit donc l'intérêt des guides de contraintes ou de déformations du dispositif objet de l'invention, qui doivent être modélisés mécaniquement et correctement dimensionnés pour maximiser les performances.

On étudie, ci-dessous, la contribution de la nature de la literie et du guide déformation à l'amplitude du BCG.

Les guides de déformation amplifient la force d'éjection sanguine générée pendant la systole et offrent la possibilité de développer une méthode de surveillance sans contact basée sur le smartphone pour l'activité cardiaque mécanique, y compris des nouveau-nés.

Des algorithmes de traitement numérique des signaux ont été développés pour détecter les battements cardiaques, le rythme cardiaque, battement par battement, et la variabilité du rythme cardiaque (HRV) dans les signaux du BCG en utilisant des méthodes du domaine temporel ou du domaine temps-fréquence. La robustesse au bruit a également été étudiée et des algorithmes spécifiques de détection des battements cardiaques ont été conçus dans le cas du BCG pédiatrique, dont l'amplitude, par rapport aux adultes, peut être environ 30 fois plus faible en raison du faible poids et de la faible force contractile cardiaque. On montre aussi que les résolutions des accéléromètres des smartphones sont suffisantes pour les utiliser pour la surveillance de BCG en néonatalogie.

Dans une première expérimentation, le capteur est basé sur un accéléromètre analogique bidimensionnel Murata SCA100T-D02 (marques déposées) avec une densité de bruit de sortie aussi faible que 14 µg/sqrtHz. Le capteur est intégré dans un boîtier en plastique ABS et relié par un câble blindé à une unité d'acquisition Biopac MP36R (marques déposées) pour le couplage, l'amplification et la puissance en courant alternatif. La sortie analogique est couplée en courant alternatif, filtrée anti-repliement et amplifiée 100 fois avant d'être numérisée à 1 kHz. Dans cette configuration, la résolution est aussi faible que 2²¹ LSB/g (bit le moins significatif).

Le processus de capture de signaux est répété pour plusieurs configurations de matelas (avec ou sans housse) et pour les couchages suivants : sans guide de déformation, ruban adhésif en polypropylène (PP), ruban de coton, tissus d'espacement. Le tableau 1 illustre toutes ces configurations. Un échantillon témoin, sans personne sur le lit, est ajouté pour mesurer la ligne de base du bruit. Chaque configuration est répétée trois fois pour éliminer la variabilité de la position du lit.

Dans la deuxième expérimentation, les positions du lit et du capteur sont les mêmes que dans l'expérience précédente. Cette fois, le capteur est basé sur un smartphone : il s'agit d'un accéléromètre numérique 3D LSM6DSM de STMicroelectronics (marques déposées), intégré dans un téléphone Motorola One (marque déposée). Dans cette configuration de smartphone, le taux d'échantillonnage est de 200 Hz, la résolution est de 2¹² LSB/g et la densité de bruit de sortie est de 90 µg/sqrtHz. On note que ces spécifications sont bien moindres que celles du capteur dans la première expérimentation. L'application Android (marque déposée) en tâche de fond Fealing est utilisée pour enregistrer les échantillons de l'accéléromètre et les exporter sur un ordinateur.

Le même adulte se couche sur le lit, immobile et en position couchée pour une sieste de 30 minutes, selon deux configurations différentes : avec le smartphone fixé par un velcro sur une literie à guide de déformation ou directement sur la housse du matelas. Un autre capteur est utilisé comme référence : le capteur de pression normale EMFIT QS (marque déposée), qui fournit un signal brut pour les siestes de plus de 20 minutes, dans les bandes de fréquence de la respiration (0,07-3 Hz) et du coeur (1-35 Hz).

La première minute est éliminée de chaque enregistrement afin de s'assurer que le volontaire est détendu et respire lentement et régulièrement pendant les enregistrements d'une minute qui en résultent. Les signaux numériques du BCG sont filtrés avec des filtres de Butterworth de troisième ordre, en particulier un filtre passe-bas de 25 Hz et un filtre passe-haut de 2 Hz. Ils sont appliqués en avant et en arrière afin d'éviter toute distorsion de phase. Enfin, le signal est décimé à une fréquence d'échantillonnage de 200 Hz.

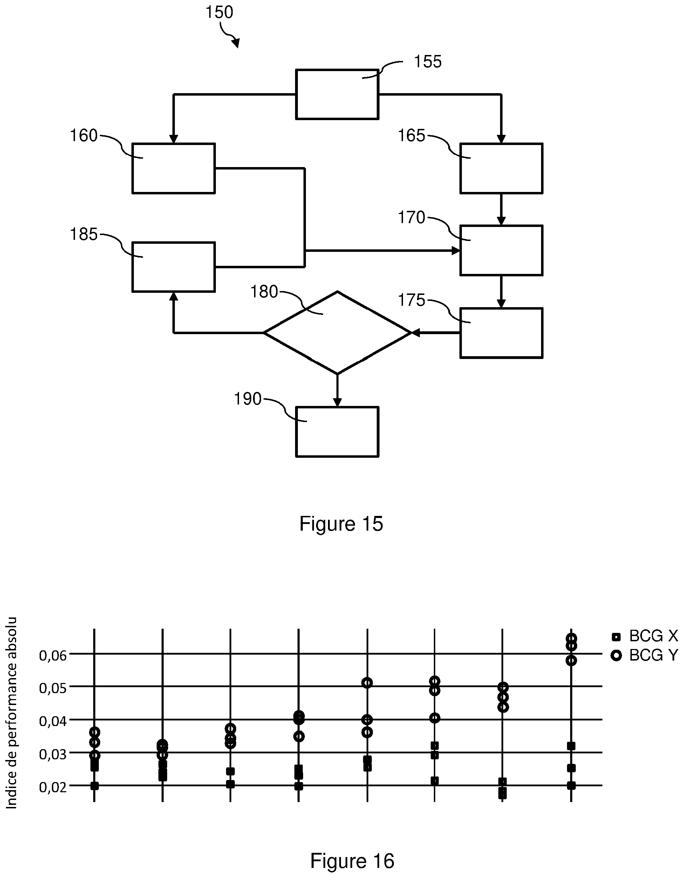

Les battements de coeur sont détectés à l'aide d'un algorithme de correspondance de gabarit de distorsion temporelle dynamique (DTW), dont les étapes sont rappelées dans la figure 15.

Les pics J du BCG sont définis comme étiquettes de référence pour les battements de coeur.

La figure 15 représente un pseudo-algorithme 150 de détection des battements cardiaques à l'aide d'une méthode d'appariement de gabarits DTW.

On observe, en figure 15, une étape 155 de segmentation du BCG sans mouvement, une étape 160 de détection automatique d'une forme ou gabarit de battement cardiaque, une étape 165 de segmentation des potentiels battements cardiaques, situés autour de certains extrema locaux du signal, une étape 170 de mesure de la distance DTW des battements candidats avec le gabarit, une étape 175 de détection des battements cardiaques, car-à-dire ceux dont la distance DTW est la plus petite, une étape 185 d'estimation de moyenne des battements cardiaques sélectionnés, pour affiner le gabarit pour les nouvelles mesures de distance avec les candidats et une étape 180 de détermination de convergence du gabarit. Tant que la convergence n'a pas eu lieu, après l'étape 180, le cycle d'étapes 185, 170, 175 et 180 se reproduit. Une fois la convergence réalisée, on passe à la détection des pics J.

L'amplitude médiane des pics J est un simple indice de performance, mais ne tient pas compte de deux phénomènes :
- la modulation de l'amplitude des J pics pendant le cycle respiratoire, comme le montre la figure 16 et
- la non-linéarité de la structure mécanique.

Par conséquent, les pics J les moins détectables doivent être amplifiés en priorité.

Le premier décile de l'amplitude des J-crêtes est sélectionné pour chaque signal BCG. L'indice de performance absolu est la valeur médiane de ces premiers déciles sur les trois enregistrements de cette configuration.

On évalue l'indice de performance sur chaque axe de déformation, de telle sorte à pouvoir comparer l'influence du guide de déformation sur chacun de ces axes.

Dans la deuxième expérimentation, les signaux du BCG sans mouvement sont segmentés et mis à zéro en moyenne. Le rapport signal/bruit (SNR) des signaux enregistrés par chaque capteur est estimé et une fonction de transfert est calculée, comme le rapport du SNR des capteurs de téléphones intelligents sur le SNR du capteur de pression de référence. Cette méthode est pertinente pour les segments plus longs et plus bruyants, car il n'est pas nécessaire de détecter et de vérifier manuellement les battements de coeur.

Le gain est évalué au rapport entre ces fonctions de transfert et peut dépendre de la fréquence, en particulier de deux bandes de fréquence : la bande de fréquence respiratoire et la bande de fréquence cardiaque, précédemment définies comme 0,07-3 Hz et 1-35 Hz. Les bandes de fréquences sont filtrées à l'aide de filtres de Butterworth de troisième ordre.

Les signaux du BCG pendant l'absence ou la présence sont nécessairement enregistrés à des intervalles de temps différents. Idéalement, les capteurs des smartphones enregistrent le BCG simultanément ; pour des raisons de simplicité, on les enregistre également à des moments différents. Au total, deux siestes sont enregistrées : une avec guide de déformation et une sans. Pour chacune de ces siestes, le BCG est segmenté en segments sans mouvement, avec ou sans présence et avec deux largeurs de bande de fréquences différentes. Les trois axes des capteurs de l'accéléromètre sont étudiés.

La conclusion de ces expérimentations est que la configuration du matelas a un impact direct sur l'indice de performance. De plus, les indices de performance sont dépendants des axes. Trois résultats principaux ressortent de la figure 16, qui classe les configurations en fonction de leurs indices de performance sur l'axe Y, avec, de gauche à droite, tissus d'espacement avec housse, ruban adhésif PP avec housse, pas de guide avec housse, pas de guide sans housse, ruban de coton avec housse, tissus d'espacement sans housse, ruban adhésif PP sans housse, ruban de coton sans housse.

Premièrement, l'axe Y transmet mieux le signal du BCG que l'axe X. Cela a été vérifié (p<0,05) pour chaque configuration, sauf pour {couverture+tissu 3D} où p = 0,053, et {couverture+bande de coton} où p = 0,171.

Deuxièmement, l'ajout d'une couverture à la configuration du matelas modifie la transmission du signal BCG le long de l'axe Y (p<0,05), sauf lorsqu'aucun guide d'onde n'est utilisé (p=0,177).

Troisièmement, le guide de déformation en ruban de coton améliore, indépendamment de la configuration du matelas, l'indice de performance le long de l'axe Y, ce qui n'est pas le cas des autres guides de déformation. Cela a été vérifié pour l'axe Y avec un matelas sans housse (p=0,001) mais pas vraiment avec un matelas avec housse (p=0,230).

Le tableau 1 résume les indices de performance pour l'axe Y, qui est l'axe qui donne les meilleurs résultats.

**Tableau 1. Indices de performance absolus/relatifs sur l'axe Y.**

| Guide de déformation | Pas de guide de déformations | Ruban adhésif PP | Ruban de coton | Tissus d'espacement |
|---|---|---|---|---|
| Sans housse de matelas | 0,040 0,0% | 0,047 17,6% | 0,063 57,4% | 0,049 23,0% |
| Avec housse de matelas | 0,034 0,0% | 0,032 -6,6% | 0,040 16,3% | 0,033 -3,6% |

On constate que le ruban de coton permet en général une meilleure transmission que les tissus d'espacement ou le ruban adhésif en PP.

## Revendications

1. Dispositif de ballistocardiographie (200, 300), **caractérisé en ce qu'**il comporte :
- un support non homogène et anisotrope (105) comportant une partie formant un guide (205, 255, 305) de contraintes ou de déformations et une partie transmettant moins de contraintes ou de déformations dans le domaine des fréquences entre 0,05Hz et 25 Hz, le guide de contraintes ou de déformations recouvrant au moins partiellement le support (105), et
- au moins un capteur (210) d'un signal représentatif d'au moins un déplacement et/ou une variation de contrainte quasi statique dudit guide dans le domaine des fréquences entre 0,05 Hz et 25 hertz, positionné en regard dudit guide de contraintes ou de déformations ;
dans lequel le guide de contraintes ou de déformations, dit « surfacique », est un élément dont une dimension est au moins dix fois inférieure par rapport à deux autres dimensions dans un repère orthonormé.

2. Dispositif de ballistocardiographie (200, 300) selon la revendication 1, dans lequel le guide (205, 255, 305) de contraintes ou de déformations présente une tension sur sa longueur.

3. Dispositif de ballistocardiographie (200, 300) selon l'une des revendications 1 ou 2, qui comporte un moyen de serrage (245) sous tension du guide (205) de contraintes ou de déformations autour du support.

4. Dispositif de ballistocardiographie (200) selon la revendication 3, dans lequel le moyen de serrage (245) ou de fixation (265, 280) ne couvre pas toute la largeur du guide (205, 255) de contraintes ou de déformations.

5. Dispositif de ballistocardiographie (200, 300) selon l'une des revendications 1 à 4, dans lequel le guide (205, 255, 305) de contraintes ou de déformations présente un module d'Young supérieur, d'au moins 10 % à la valeur du module d'Young en dehors du guide de contraintes ou de déformations selon au moins une direction.

6. Dispositif de ballistocardiographie (200, 300) selon l'une des revendications 1 à 5, dans lequel le support (105) présente une forme généralement parallélépipédique dont la plus grande dimension est dénommée «longueur» (225), la plus petite dimension est dénommée «épaisseur» (230) et la dimension intermédiaire est dénommée «largeurs (220) et au moins un capteur (210) est un capteur d'une inclinaison du guide de contraintes (205, 305) ou de déformations et ce guide est positionné selon une direction parallèle à la largeur et passant par une source de contraintes ou de déformations.

7. Dispositif (200, 300) selon l'une des revendications 1 à 6, dans lequel le guide de contraintes ou de déformations présente un module d'Young de valeur progressive.

8. Dispositif (200, 300) selon l'une des revendications 1 à 7, dans lequel le guide de contraintes (205, 305, 410) ou de déformations comporte au moins un matériau tissé.

9. Dispositif (200, 300) selon l'une des revendications 1 à 8, qui comporte, de plus, un moyen de traitement (215) de chaque signal capturé par chaque capteur (210) et un moyen de comparaison (235) à au moins un modèle prédéterminé pour en déduire des tendances, des troubles ou des anomalies.

10. Procédé de ballistocardiographie (600) mettant en oeuvre un dispositif (200, 300) selon l'une des revendications 1 à 9, **caractérisé en ce qu'**il comporte les étapes suivantes :
- capture (601) d'un signal représentatif d'un déplacement ou d'une variation de contrainte quasi statique produite par un utilisateur et parcourant un support,
- segmentation (603 à 608) du signal capturé,
- filtrage (609) d'au moins un segment du signal capturé fournissant un signal représentatif d'une activité cardiaque comportant au moins deux battements cardiaques,
- application (612) d'un modèle (613) à chaque période du signal représentatif d'une activité cardiaque et
- détermination (614) d'une fréquence cardiaque et/ou d'une variabilité de la fréquence cardiaque.

11. Procédé (600) de ballistocardiographie selon la revendication 10, qui comporte une phase de calibrage (700) du modèle qui comporte les étapes suivantes :
- capture (701) d'un signal représentatif d'un déplacement ou d'une variation de contrainte quasi statique produite par un utilisateur et parcourant un support,
- segmentation (703 à 712) du signal capturé,
- détection (714) d'une enveloppe et d'au moins une période pour chaque segment de signal,
- calcul (716 et 717) d'un centre de chaque enveloppe dans la période,
- superposition (715) de centres de chaque période et
- pour chaque segment du signal, création d'un modèle (718) cardiaque correspondant à la moyenne des points superposés à chaque instant de la période prédéterminée.

12. Procédé (600) de ballistocardiographie selon l'une des revendications 10 ou 11, dans lequel l'étape de filtrage (609) fournit un signal représentatif d'une activité respiratoire, le procédé comportant, de plus, une étape de détermination (611) d'une fréquence respiratoire et/ou d'événement d'apnée/dyspnée en fonction d'au moins un segment de signal représentatif d'une activité respiratoire.

13. Procédé (600) de ballistocardiographie selon l'une des revendications 10 à 12, dans lequel l'étape de segmentation comporte une étape d'élimination (604, 607) de chaque segment de signal représentatif d'un mouvement de l'utilisateur et/ou d'une absence de l'utilisateur sur le support.

## Patentansprüche

1. Ballistokardiographievorrichtung (200, 300), **dadurch gekennzeichnet, dass** sie aufweist:
- einen nicht homogenen und anisotropen Träger (105) mit einem Teil, der eine Belastungs- oder Verformungsführung (205, 255, 305) bildet, und einem Teil, der weniger Belastungen oder Verformungen im Frequenzbereich zwischen 0,05 Hz und 25 Hz überträgt, wobei die Belastungs- oder Verformungsführung den Träger (105) mindestens teilweise bedeckt, und
- mindestens einen Sensor (210) eines Signals, das für mindestens eine Bewegung und/oder quasistatische Belastungsänderung der Führung im Frequenzbereich zwischen 0,05 Hz und 25 Hertz repräsentativ ist, welcher der Belastungs- oder Verformungsführung zugewandt positioniert ist;
wobei die als "oberflächlich" bezeichnete Belastungs- oder Verformungsführung ein Element ist, dessen Abmessung im Verhältnis zu zwei anderen Abmessungen in einem orthonormierten Bezugssystem mindestens zehnmal kleiner ist.

2. Ballistokardiographievorrichtung (200, 300) nach Anspruch 1, wobei die Belastungs- oder Verformungsführung (205, 255, 305) über ihre Länge eine Spannung aufweist.

3. Ballistokardiographievorrichtung (200, 300) nach einem der Ansprüche 1 oder 2, die ein Klemmmittel (245) unter Spannung der Belastungs- oder Verformungsführung (205) um den Träger umfasst.

4. Ballistokardiographievorrichtung (200) nach Anspruch 3, wobei das Klemm- (245) oder Befestigungsmittel (265, 280) nicht die gesamte Breite der Belastungs- oder Verformungsführung (205, 255) bedeckt.

5. Ballistokardiographievorrichtung (200, 300) nach einem der Ansprüche 1 bis 4, wobei die Belastungs- oder Verformungsführung (205, 255, 305) ein Young-Modul aufweist, das mindestens 10 % höher ist als der Wert des Young-Moduls außerhalb der Belastungs- oder Verformungsführung in mindestens einer Richtung.

6. Ballistokardiographievorrichtung (200, 300) nach einem der Ansprüche 1 bis 5, wobei der Träger (105) eine allgemein parallelepipedische Form aufweist, deren größte Abmessung als "Länge" (225), die kleinste Abmessung als "Dicke" (230) und die Zwischenabmessung als "Breite" (220) bezeichnet wird und mindestens ein Sensor (210) ein Sensor einer Neigung der Belastungs- oder Verformungsführung (205, 305) ist und diese Führung in einer Richtung positioniert ist, die parallel zur Breite ist und durch eine Belastungs- oder Verformungsquelle verläuft.

7. Vorrichtung (200, 300) nach einem der Ansprüche 1 bis 6, wobei die Belastungs- oder Verformungsführung ein Young-Modul mit progressivem Wert aufweist.

8. Vorrichtung (200, 300) nach einem der Ansprüche 1 bis 7, wobei die Belastungs- oder Verformungsführung (205, 305, 410) mindestens ein Gewebematerial aufweist.

9. Vorrichtung (200, 300) nach einem der Ansprüche 1 bis 8, die weiterhin ein Verarbeitungsmittel (215) jedes von jedem Sensor (210) erfassten Signals und ein Vergleichsmittel (235) mit mindestens einem vorbestimmten Modell umfasst, um daraus Trends, Störungen oder Anomalien abzuleiten.

10. Ballistokardiographieverfahren (600), das eine Vorrichtung (200, 300) nach einem der Ansprüche 1 bis 9 verwendet, **dadurch gekennzeichnet, dass** es die folgenden Schritte aufweist:
- Erfassung (601) eines Signals, das für eine Bewegung oder eine quasistatische Belastungsänderung repräsentativ ist, die von einem Benutzer erzeugt wird und einen Träger durchläuft,
- Segmentierung (603 bis 608) des erfassten Signals,
- Filtern (609) mindestens eines Segments des erfassten Signals, wodurch ein Signal bereitgestellt wird, das für eine Herzaktivität mit mindestens zwei Herzschlägen repräsentativ ist,
- Anwendung (612) eines Modells (613) auf jede Periode des Signals, das für eine Herzaktivität repräsentativ ist, und
- Bestimmung (614) einer Herzfrequenz und/oder einer Herzfrequenzvariabilität.

11. Ballistokardiographieverfahren (600) nach Anspruch 10, das eine Kalibrierphase (700) des Modells umfasst, die folgende Schritte umfasst:
- Erfassung (701) eines Signals, das für eine Bewegung oder eine quasistatische Belastungsänderung repräsentativ ist, die von einem Benutzer erzeugt wird und einen Träger durchläuft,
- Segmentierung (703 bis 712) des erfassten Signals,
- Erkennung (714) einer Hülle und mindestens einer Periode für jedes Signalsegment,
- Berechnung (716 und 717) eines Zentrums jeder Hülle in der Periode,
- Überlagerung (715) von Zentren jeder Periode und
- für jedes Segment des Signals Erstellung eines Herzmodells (718), das dem Mittelwert der überlagerten Punkte zu jedem Zeitpunkt der vorbestimmten Periode entspricht.

12. Ballistokardiographieverfahren (600) nach einem der Ansprüche 10 oder 11, wobei der Filterschritt (609) ein repräsentatives Signal einer Atemaktivität liefert, wobei das Verfahren weiterhin einen Schritt (611) zur Bestimmung einer Atemfrequenz und/oder eines Apnoe-/Dyspnoe-Ereignisses in Abhängigkeit von mindestens einem repräsentativen Signalsegment einer Atemaktivität aufweist.

13. Ballistokardiographieverfahren (600) nach einem der Ansprüche 10 bis 12, wobei der Segmentierungsschritt einen Schritt zur Entfernung (604, 607) jedes Signalsegments aufweist, das für eine Bewegung des Benutzers und/oder eine Abwesenheit des Benutzers auf dem Träger repräsentativ ist.

## Claims

1. Ballistocardiography device (200, 300, 400), **characterised in that** it comprises:
- a non-homogeneous anisotropic support (105) having a portion forming a stress or deformation guide (205, 255, 305) and a portion transmitting fewer stresses or deformations in the frequency range between 0.05 Hz and 25 Hz, the stress or deformation guide covers the support (105) at least partially; and
- at least one sensor (210) of a signal representing at least one movement and/or variation of quasi-static stress of the guide in the frequency range between 0.05 Hz and 25 Hz, positioned facing the stress or deformation guide,
wherein the stress or deformation guide, referred to as "surfacic", is an element for which one dimension is at least ten times less than two other dimensions in an orthonormal reference space.

2. Ballistocardiography device (200, 300) according to claim 1, wherein the stress or deformation guide (205, 255, 305) is tensioned along its length.

3. Ballistocardiography device (200, 300) according to one of claims 1 or 2, which comprises a means (245) for tightening under tension the stress or deformation guide (205) around the support.

4. Ballistocardiography device (200) according to claim 3, wherein the means for tightening (245) or fastening (265, 280) does not cover the entire width of the stress or deformation guide (205, 255).

5. Ballistocardiography device (200, 300) according to one of claims 1 to 4, wherein the stress or deformation guide (205, 255, 305) has a Young's modulus at least 10% higher than the value of the Young's modulus outside the stress or deformation guide in at least one direction.

6. Ballistocardiography device (200, 300) according to one of claims 1 to 5, wherein the support (105) has a generally parallelepiped shape, whose largest dimension is called the "length" (225), smallest dimension is called the "thickness" (230), and intermediate dimension is called the "width" (220), and at least one sensor (210) is a sensor for capturing an inclination of the stress or deformation guide (205, 305), and this guide is positioned in a direction parallel to the width and passing through a source of stresses or deformations.

7. Device (200, 300) according to one of claims 1 to 6, wherein the stress or deformation guide has a Young's modulus with a progressive value.

8. Device (200, 300) according to one of claims 1 to 7, wherein the stress or deformation guide (205, 305, 410) comprises at least one woven material.

9. Device (200, 300) according to one of claims 1 to 8, which further comprises a means (215) for processing each signal captured by each sensor (210), and a means (235) for comparing to at least one predefined model in order to deduce patterns, disorders or defects from this.

10. Ballistocardiography method (600) utilising a device (200, 300) according to one of claims 1 to 9, **characterised in that** it comprises the following steps:
- capturing (601) a signal representative of a movement and/or variation of quasi-static stress produced by a user and traversing a support;
- segmenting (603 to 608) the captured signal;
- filtering (609) at least one segment of the captured signal providing a signal representative of a cardiac activity comprising at least two heartbeats;
- applying (612) a model (613) to each period of the signal representative of a cardiac activity; and
- determining (614) a heart rate and/or a heart rate variability.

11. Ballistocardiography method (600) according to claim 10, which comprises a phase (700) of calibrating the model, which comprises the following steps:
- capturing (701) a signal representative of a movement and/or variation of quasi-static stress produced by a user and traversing a support;
- segmenting (703 to 712) the captured signal;
- detecting (714) an envelope and at least one period for each signal segment;
- calculating (716 and 717) a centre of each envelope in the period;
- superimposing (715) centres of each period; and
- for each segment of the signal, creating (718) a cardiac model corresponding to the mean of the superimposed points at each instant of the predefined period.

12. Ballistocardiography method (600) according to one of claims 10 or 11, wherein the filtering step (609) supplies a signal representative of a respiratory activity, the method also comprising a step (611) of determining a respiratory frequency and/or apnoea/dyspnoea events as a function of at least one signal segment representative of a respiratory activity.

13. Ballistocardiography method (600) according to one of claims 10 to 12, wherein the segmentation step comprises a step (604, 607) of removing each signal segment representative of a movement by the user and/or an absence of the user on the support.
